# EUROPEAN PATENT SPECIFICATION

(11) **EP 1 600 514 B1**
(45) Date of publication and mention of the grant of the patent: **31.08.2011**
(21) Application number: 04701340.4
(22) Date of filing: 12.01.2004
(51) Int. Cl.: C12Q 1/14

(54) **SELECTIVE CULTURE MEDIUM FOR THE ISOLATION AND/OR DETECTION OF SPECIES IN THE STREPTOCOCCUS GENUS**
SELEKTIVES KULTURMEDIUM ZUR ISOLATION UND/ODER DETEKTION VON SPEZIES DES STREPTOCOCCUS GENUS
MILIEU DE CULTURE SELECTIF POUR L'ISOLEMENT ET LA DETECTION D'ESPECES DU GENRE STREPTOCOCCUS

(30) Priority: 10.01.2003 CU 803
(43) Date of publication of application: 30.11.2005
(73) Proprietor: CENTRO NACIONAL DE BIOPREPARADOS, Bejucal, Provincia La Habana 6048 (CU)
(72) Inventor: DURAN VILA, Anabel, 10 de Octubre, 10500 Ciudad de la Habana (CU); RODRIGUEZ MARTINEZ, Claudio, 6048 La Habana (CU)
(74) Representative: Winckels, Johannes Hubertus F.
(86) International application number: PCT/CU2004/000001
(87) International publication number: WO 2004/063392

(56) References cited:
- FR-A- 2 728 587
- US-A- 4 259 442
- US-A- 4 532 206
- US-A- 5 464 755
- US-A- 5 620 865
- GRANATO P A ET AL: "A new medium for the presumptive identification of gram-positive uropathogens." HEALTH LABORATORY SCIENCE. OCT 1976, vol. 13, no. 4, October 1976 (1976-10), pages 258-261, XP008033254 ISSN: 0017-9035
- LITTEL K J ET AL: "FLUOROGENIC SELECTIVE AND DIFFERENTIAL MEDIUM FOR ISOLATION OF FECAL STREPTOCOCCI" APPLIED AND ENVIRONMENTAL MICROBIOLOGY, WASHINGTON,DC, US, vol. 45, no. 2, 1 February 1983 (1983-02-01), pages 622-627, XP000568357 ISSN: 0099-2240
- MANAFI M: "New developments in chromogenic and fluorogenic culture media" INTERNATIONAL JOURNAL OF FOOD MICROBIOLOGY, ELSEVIER SCIENCE PUBLISHERS, AMSTERDAM, NL, vol. 60, no. 2-3, 2000, pages 205-218, XP002177549 ISSN: 0168-1605 cited in the application
- CARRICAJO A ET AL: "Comparative evaluation of five chromogenic media for detection, enumeration and identification of urinary tract pathogens" EUROPEAN JOURNAL OF CLINICAL MICROBIOLOGY AND INFECTIOUS DISEASES, vol. 18, no. 11, November 1999 (1999-11), pages 796-803, XP001197271 ISSN: 0934-9723 cited in the application

## Description

### TECHNICAL SECTOR

The present invention is related with Microbiology and specifically with the clinical and veterinary diagnosis, with the food quality control and with the environmental contamination studies where the methods for the isolation and detection of *Streptococcus* species are needed.

### PRIOR ART

The streptococci are considered as a fastidious microorganism genus whose identification is extremely important in the clinical diagnosis. Within the classification carried out by Lancefield there are numerous pathogenic species to human, such as: *Streptococcus pyogenes*, *Streptococcus agalactiae, Streptococcus anginosus*, *Enterococcus avium, Enterococcus faecium, Enterococcus faecalis,* causal agents of septicemia, neonatal meningitis, endocarditis and urinary tract infections (Giuseppe, N. and Vito Mar N., 1989. Diccionario de Bacteriologia Humana, edición española Menarini). Although the streptococci aren't the microorganisms that more frequently cause urinary sepsis, it is necessary to detect them, because specifically hemolytic streptococci are the most frequent cause of neonate sepsis, so its early detection allows to administer intraparto antibiotic prevention and to prevent the perinatal infection by this microorganism (Bosch, J.; Murillo, S.; Rico, M. y Salgado, M. 1998. Utilidad de un medio selectivo disco-caldo para la detección de estreptococo del grupo B en la vagina. Enferm. Infecc. Microbiol. Clin. Vol 16(2)). The early incidence of neonatal sepsis is of 1.48-4.08/1,000 alive born children with a mortality of 8.7-10.5% (Montero, R.; Barbadillo, F.; Anso, S.; Marrero, M.; Carpintero I.; Sastre E. y Alonso B.: Sepsis neonatal por *Streptococcus agalactiae.* Qué hacer ?. An-Esp-Pediatr. 1998; 48 (3), p. 288-92; Juncosa, T.; Bosch, J.; Dopico, E.; Guardia, C.; Lite, J.; Sierra, M.; Barranco, M.; Matas, L.; Sanchez, F.; Sanfeliu, I. and Vinas, L. 1998. Infección neonatal por *Streptococcus agalactiae.* Estudio multicéntrico en el área de Barcelona. Enferm-Infecc-Microbiol-Clin.; 16(7), p. 312-5). The causal agent of this illness is mostly *Streptococcus agalactiae,* habitual resident of the female genital tract. Overmore new, not known urinary pathogens, as *Streptococcus urinalis* sp.nov., also appears, that although have similarity with *Streptococcus pyogenes* and *Streptococcus canis.* It is considered a new species of *Streptococcus* genus and it's taxonomical denomination is *Streptococcus urinalis* sp.nov., strain type CCUG 41590T (Collins, M.D.; Hutson, R.A.; Falsen, E.; Nikolaitchouk, N.; La Claire, L. and Facklam, R.R. 2000. An unusual Streptococcus from human urine, Streptococcus urinalis sp. nov. Int J Syst Evol Microbiol; 50 Pt 3, p. 1173-1178).

Actually, there are in the market many culture media that are designed to isolate and/or to detect different species of the *Streptococcus* genus. Among them can be mentioned: Blood Agar, Blood Columbia Agar, Tryptone Soja Agar with Blood, Brain Heart Agar, and other (Manual of cultivation Means OXOID. 1995. UNIPATH, España; MERCK Microbiology Manual. 2000. Merck KgaA, Darmstadt; Manual DIFCO de Bacteriologia. 1984. Décima Edición, Francisco Soria Melguizo, S.A., Madrid).

These non-selective media do not allow the differentiation between pathogenic and no-pathogenic species. These media are not selective and allow the growth of other Gram- positive and Gram-negative bacteria that compete with the streptococci for the nutrients. In case of Proteus, for example, the strains that develop "spread" impede the isolation of the target colonies.

There are no highly nutrient substances in their formulations, thus the blood addition is necessary which hinders the manipulation and increases the risk of contamination. In addition it doesn't allow an extended expiration date of the medium, because the blood is biologically unstable compound.

The CLED medium, offered by the previously mentioned firms, is used widely in the laboratories and it is considered as one of the best options. It allows the growth of all urinary pathogens, *Streptococcus* among them, and other Gram-positive microorganisms that cannot be differentiated, because they possess the same cultural characteristics. *Streptococcus* growth is slow. The enterococci (*Enterococcus faecalis, Enterococcus faecium* and *Enterococcus avium*) can be confused with *Shigella* species.

In the MERCK Manual of culture media (MERCK Microbiology Manual, 2000. Merck KgaA, Darmstadt) an agar medium and a broth are described for the selective enrichment of *Streptococcus* that uses sodium azide and sodium sulfite as inhibitors of Gram-negative organisms. As disadvantages of these media can be mentioned their high toxicity by the presence of the sodium azide (highly toxic ingredient) and the impossibility of differentiating between the species in the broth, which is used specifically to promote the microbial growth.

The OXOID manual (Manual of culture media, OXOID, 1995. UNIPATH, Spain) describes a selective medium for *Streptococcus* that requires the addition of blood and a selective supplement that contains colistin sulfate and oxolinic acid. However, it is also indispensable to incubate in a 5 % CO2 atmosphere. Together with the previously mentioned difficulties, it needs the addition of blood, the use of antibiotics as a supplement. Moreover, it makes the preparation of the medium difficult and it doesn't guarantee a real inhibition of the undesirable accompanying microbiota due to the appearance, every time with more frequency, of resistance phenomenons to the antibiotics, mainly in the Gram negative organisms.

Furthermore, the necessity to incubate the medium in 5 % CO2 atmosphere makes the isolation technique bothersome, when this method isn't directed to the identification or differentiation, so it is necessary to use culture media or biochemical and serological tests to identify *Streptococcus* target species.

In the before mentioned Manual, the composition of a *Streptococcus*/*Staphylococcus* selective medium that needs the addition of sterile blood and the antibiotic supplement, nalidixic acid and colistin sulfate is also described. This formulation employment presents the same above-mentioned disadvantages, relative to the use of blood and antibiotics.

A selective medium for the rapid isolation of *Streptococcus agalactiae* and other streptococci related with the bovine mastitis: is Edwards (Modified) medium. It is described in the OXOID Manual 1995. It is a blood supplemented medium and it contains talium sulfate and crystal violet as inhibitors. This diagnostic medium allows the growth of *Streptococcus agalactiae* and other *Streptococcus,* using like identification principle only the esculin hydrolysis. The D group streptococci form a precipitate in the medium (black colonies) due to its metabolic activity and the other microorganisms give the negative answer (blue colonies) and cannot be differentiated.

The crystal violet used as a selective agent, can inhibit the growth of *Streptococcus* cell. The talium sulfate is also considered a highly toxic compound, noxious for the human health and extremely harmful for the environment. Subsequently it makes at the same time the production process, preparation and discarding of the medium wastes very bothersome.

The Todd-Hewitt medium with gentamicin and nalidixic acid, Colistin Blood Agar with colistin and nalidixic acid, Mueller-Hinton Broth with 5% of serum and amikacin (Bosch, J.; Murillo, S.; Rico, M. and Salgado, M. 1998. Utilidad de un medio selectivo disco-caldo para la detección de estreptococo del grupo B en la vagina. Enferm. Infecc. Microbiol. Clin. 16(2)) and the Azide-Esculin-Oxolinic acid medium has been used already for the detection and identification of specific *Streptococcus* species. They have the similar to the previously mentioned disadvantages (Figueras, M.J; Inza, I.; Polo, F. and Guarro, J. 1998. Evaluation of the oxolinic acid-esculin-azide medium for the isolation and enumeration of faecal streptococci in a routine monitoring programme for bathing waters. Can. J. Microbiol. 44, p. 998-1002).

There have been patens and patent applications directed to the culture of specific *Streptococcus* species. Some of them are related below.

De la Rosa Fraile describes a dehydrated medium to identify group B *Streptococcus* in the EP 1 098 002 A1 patent presented in 2001.05.09 (Dehydrated immediate reconstruction culture medium to identify group B Streptococci (*Streptococcus agalactiae*) by detection of their pigment), where the differentiation is done by its own pigment production. The essence of the invention consists on adding Sephadex 200 as gelling agent to the Granada medium.

The limitations of the mentioned invention can be summarized underneath:
It allows only the identification of *Streptococcus agalactiae.*
It is needed the addition of horse serum.
The prepared medium has a durability of less than 2 months due to the unstableness of the compounds.
It is necessary to supplement antibiotics such as metronidazole and colistin, that already presents the deficiencies similar to those described for the previous formulations.
The cultural characteristics of the microorganism cannot be observed, because the medium is semisolid.

The preparation is complex, and it includes different steps, such as the preparation of the basal medium, the addition of the gel, the agitation to catch bubbles, the addition of the supplements and the incubation au bain-marie.

Years before the same author developed a formulation for the detection of *Streptococcus agalactiae* (De La Rosa Fraile, Manuel; Patent: ES 2088827, 1996.09.16. Culture medium for *Streptococcus agalactiae* which activates the formation of pigment by amylases and/or (G((alpha 1-4)G) (n(2) glucooligosaccharides added as chemical compounds, as polysaccharide hydrolysates or generated in the same medium using enzymes and polysaccharides) that contained antibiotics in supplement form, amylases, malto-oligosaccharides, starch for the production of the characteristic pigment of this microorganism. This formulation is complex to prepare, it contains labile enzymes added as supplement and complex chemical and labile compounds (malto-oligosaccharides). Therefore, its employment in the culture media production is not very common.

Another medium designed to the identification of group B hemolytic *Streptococcus,* is based on the production of a color orange pigment. It was developed by Tanaka Yoshihiro and Takahashi Hisayoshi (JP 9313171A2, 1997.12.09. Culture medium for group B hemolytic *Streptococcus*). The medium contains glucose, sodium piruvate, meat peptone, MgSO4, MOPS-phosphate, methotrexate and antibacterial agent (inactive for group B *Streptococcus*). By use of this formulation, other streptococci cannot be identified.

In the patent JP 59159797 (Kojima, H., et to the one. 1984.09.10. Culture medium for selective proliferation of Streptococcus hemolyticus), a selective medium for the propagation of *Streptococcus hemolyticus* is described. It contains lipids and fatty acids as specific selective agents to promote the growth of this specie. The employment of the soja bean in this medium makes complex its preparation, as well as the selective agents don't inhibit the growth of other target *Streptococcus* species.

Deyloff John protected a culture medium for the differentiation of *Streptococcus mutans* (US 4468456, 1984.08.28. Medium for differentiating *Streptococcus mutans*) composed by phosphate salts, agar, yeast extract, sucrose and a pH indicator. The product preparation is complicated since it is composed of two solidified layers. To one of which it is necessary to add calcium phosphate suspension, which is difficult to disolve. The medium is not sufficiently inhibitory for other Gram-positive species.

Other media and methods have been developed for the culture of specific *Streptococcus* species to obtain certain metabolites, or even, for the diagnosis. This products in any case offers the possibility of differentiation or detection of a significant number of *Streptococcus* species. Among them can be mentioned Park et al. and Inoue et al. (US 5496726, 1996. *Streptococcus zooepidemicus* medium and process for preparing hyaluronic acid; US 4 306 024, 1981.12.15. Process for cultivation of hemolytic *Streptococcus pyogenes*).

Blareau Jean Pierre and collaborators (Patente: FR 2723960-A1, 1994.08.31. Cultures of Streptococcus thermophilus to activite beta-galactosidase elevee, leur proceeds d'obtention, et leurs utilisations) patented the culture medium for Streptococcus thermophilus that contains only milk protein hydrolysates, lactose and yeast extract. The medium isn't designed to the clinical diagnosis, neither to the control of the food quality and isn't selective medium for the Streptococcus detection.

Another group of inventions contrarily is directed to the culture of several genera or Streptococcus species, and don't allow a differentiation or appropriate identification among them, various examples make are cited next:

In the last years, there was an increase in the use of chromogenic and fluorogenic substrates that have been incorporated to the traditional media or in the new culture media, developed specifically for some microorganisms according to its metabolic demands. These chromogenic reactions possess certain advantages regarding to the conventional media, such as rapidity in obtaining of results, high specificity and sensibility due to the specific enzyme substrate degradation.

Manafi in various articles and extensive reviews mentioned the chromogenic and fluorogenic substrates more frequently employed as compounds of the culture media. In addition, he explains its possible usages in the identification of some species of *Streptococcus.* In the article "New developments in chromogenic and fluorogenic culture media" (Manafi, M. 2000. International Journal of Food Microbiology 60, p. 205-218), for example, he refers to some of these media, which present certain limitations or disadvantages:
- ■: Enterolert (IDEXX Laboratories Inc., Westbrook, Maine) and the Microtiter plate MUST (Sanofi, France) that contain specific fluorogenic substrates for the D-glucosidase, designed for the enterococci detection in water. Although it doesn't allow the diferentiation and identification of other streptococci and contain as a specific enzyme marker only fluorogenic substrate.
- ■: The agar was designed for the enterococci enumeration in sea water or fresh water. It contains nalidixic acid, cycloheximide, triphenyltetrazolium chloride and indoxyl beta D glucoside as a substrate. The disadvantage of this medium is the use of antibiotics as selective agents, as well as possessing of a single enzymatic marker that disables the identification of other Streptococcus. There Long incubation periods (24-48 hours) are necessary and the sensitivity of the medium is not too high.
- ■: The modification of this medium by reducing the triphenyltetrazolium chloride concentration and increasing the indoxyl beta D glucoside concentration reduced the incubation period to 24 hours but it didn't increase the diagnostic possibilities of the medium.
- ■: Chromocult enterococci broth (CEB) (BBL) and the Readycult enterococci (MERCK) use the 5-bromo-4-chloro-3-indolyl- beta D glucopyranoside as the substrate to identify Enterococcus and the sodium azide to inhibit other microorganisms. However, there appear false positive results in the medium, such as Leuconostoc, Lactococcus lactis and Aeromonas sp. Other streptococci are not identified and the medium contains the toxic ingredients.

The CHROMagar Orientation medium (BBL) allows the presumptive simultaneous identification of Gram positive, Gram negative organisms and yeasts (Carricajo, A.; Boiste, S.; Thore, J.; Aubert, G.; Gille, Y and Freydiere, AM. 1999. Comparative evaluation of five chromogenic media for detection, enumeration and identification of urinary tract pathogens. Eur J Clin Microbiol Infect Dis 18(11), p. 796-803; Samra, Z.; Heifetz, M.; Talmor, J.; Bain, E. and Bahar, J. 1998. Evaluation of use of a new chromogenic agar in detection of urinary tract pathogens. J Clin Microbiol., 36(4), p. 990-994; Hengstler, KA; Hammann, R. and Fahr, AM. 1998. Evaluation of BBL CHROMagar orientation medium for detection and presumptive identification of urinary tract pathogens. J Clin Microbiol. 35(11), p.2773; Merlino, L; Siarakas, S.; Robertson, GJ.; Funnell, GR.; Gottlieb, T and Bradbury, R. 1997. Evaluation of CHROMagar Orientation for differentiating and presumptive identification of gram-negative bacilli and Enterococcus species. J Clin Microbiol 34(7), p.1788). Among the microorganisms that can be identified there are included the streptococci (groups D, B and C), although the groups B and C cannot be differentiated, so additional biochemical tests are required for their identification. The great diversity of colors makes the interpretation of the results extremely difficult and in occasions it is required to pay a special attention to the morphology, size and characteristics of the colonies (shine or opacity) that hinders even more the interpretation of the results. The similarity of colors of some streptococci with microorganisms of the genera Klebsiella, Enterobacter and Citrobacter, force to carry out additional tests that require more hours for carrying out. It is also prominent that the medium is sensitive to the light that changes the original color, also the incubation and reading should be made immediately.

The UTI medium (OXOID Inc.) is designed for the presumptive identification and differentiation of urinary tract infection causing microorganisms. It contains the chromogenic substrate x-glu for the enterococci identification. This formulation presents as disadvantages that other streptococci cannot be identified, other Gram negative microorganisms grow that are presented generally in urinary infections in higher concentrations than *Streptococcus* and its use is restricted to urine samples.

In 1999, Alain Rambach patented a method for the identification of microorganisms with at least two chromogenic substrates (US 5 962 251, 1999.10.05 "Method for the identification of microorganisms with at least two chromogens"). The medium contains agar, peptone, sodium chloride, yeast extract, meat extract and carbohydrates (glucose), as well as chromogenic indoxyl derived substrates (bromo-indoxyl, chloro-indoxyl, di-chloro-indoxyl, chloro-bromo-indoxyl, among other). Its main utility is the detection of presence or absence of yeasts belonging to genus *Candida,* especially *Candida albicans* and *Candida tropicalis,* although microorganisms of other genera, as well Gram-positive as Gram-negative grow with different color tonalities, and the streptococci among them. However, the diversity of developing organisms like in the previous case, delays an appropriate interpretation of the results. There is a great competition for the susbtrates, so the streptococci can be inhibited and the differentiation among other species of *Streptococcus* (non-group D) becomes impossible.

Another group of chromogenic culture media developed for the detection, enumeration and identification of urinary pathogens that allows the identification specifically D group *Streptococcus* includes CPS ID2 (bioMerieux, France), Uriselect 3 (Sanofi Diagnostic) and Rainbow UTI (Biolog, USES) (Carricajo et to the., 1999. Comparative evaluation of five chromogenic media for detection, enumeration and identification of urinary tract pathogens. Eur J Clin Microbiol Infect Dis 18(11), p. 796-803; Navarro et al., 1996. Evaluation of chromogenic medium CPS ID2 (bioMerieux) in urine cultures. Enferm Infecc Microbiol Clin Apr; 14(4), p. 215-9; Reisner and Austin, 1997. Evaluation of CPS ID2 chromogenic agar as a single medium for urine culture. Diagn Microbiol Infect Dis Jul; 28(3), p. 113-7; Willinger and Manafi, 1995. Evaluation of a new chromogenic agar medium for the identification of urinary tract pathogens. Lett Appl Microbiol May 20(5), p. 300-2) and others (Bochner B. US 5 464 755. 1995.10.07. Microbiological medium and method of assay; Yagupsky et al., 2000. Clinical evaluation of a novel chromogenic agar dipslide for diagnosis of urinary tract infections. Eur J Clin Microbiol Infect Dis 19, p. 694-698).

Chen, Chun-Ming and collaborators requested a patent of a method and medium for the detection of vancomycin-resistant enterococci (Chen, Chun-Ming; et to the one. Patent Application US 20020132285-A1. 2002.09.19. Method and medium for detecting vancomycin-resistant enterococcus). The invention consists of the use of an ingredient that produces a reaction when being metabolized by the microorganisms, in this case the enzymes beta -D-glucosidase and the pyrrolidonyl - arylamidase. It should be pointed out that the medium is unable to identify neither vancomycin sensitive enterococci nor other species of *Streptococcus.* Its preparation is troublesome, because it needs to be suplemented with antibiotics and the vancomycin doesn't inhibit some Gram-negative bacteria that can grow in the medium. Additional tests are required for the species identification.

In the application of United States patent number 20020086278-A1. 2002.06.04, Gosnell and collaborators (Chromogenic half containing blood or hemin) describe a medium that contains blood or hemin and also chromogenic substrates such as: magenta, x-glucoside, x-glucuronide, Mag-phosphate, among others. The medium allows the growth and the identification of different species by morphological characteristic, the appearance of the diverse color tonalities, fluorescence and the hemolytic reactions. The invention can be considered as the nearest prototype and the medium presents the following disadvantages:
- •: The presence of blood or hemin makes difficult the right interpretation of the chromogenic reactions. Moreover, the composition doesn't permit the inhibition of Gram-negative bacteria, among which there are a great diversity of microorganisms with enzymatic activity similar to the *Streptococcus* one.
- •: The stability period of the medium is limited, because the presence of biological compounds (blood or hemin), and its preparation is annoying.
- •: This culture medium does not contain inhibitory agents, isn't a selective medium, so it permits the development of other Gram-positive and Gram- negative genera that compete for the nutrients, and as the consequence the target microorganisms (*Streptococcus*) can't grow appropriately.
- •: The use of supplemented blood and hemin makes complex the preparation of this medium and increases the risk of its contamination.
- •: It is necessary the use of additional biochemical tests for the far along differentiation of *Streptococcus,* that needs an additional time to achieve an appropriate identification of the target germs. Specifically it is necessary to carry out an additional PYR test to differentiate *Streptococcus pyogenes* of the D group *Streptococcus* (*Enterococcus*)*.*
- •: For the identification of some microorganisms, it is necessary to keep in mind the morphology, the size and other characteristics of the colonies that are very variable because they depend on the sample type, initial concentration of the microorganism and accompanying microbiota, among other factors.
- •: The possibility that microorganisms of other genera grow like *Proteus* whose fundamental characteristic is the spread formation in the medium, makes impossible the identification of the colonies.
- •: Other microorganisms that have protein hydrolitic capacity can hydrolize the albumin and to interfere in the identification.
- •: For the preparation of the medium, chromogenic substrates can be added to the fresh medium, prepared aseptically, since some of them are labiles to high temperatures, that makes complex their preparation.
- •: The solutions of the chromogenic substrates, in some cases, form insoluble precipitate that may be more unstable after one-week storage that limits the period of usefulness of "in vitro" diagnostic.
- •: When it is used like base for Chocolate Agar, the medium is incubated in atmosphere of 5 % CO2.
- •: Some chromogenic substrates need to be dissolved in the organic solvent dimethyl sulfoxide (DMSO) that it makes the preparation of the medium troublesome.
- •: In some cases (Example 3) the D group streptococci takes the same color of other microorganisms (blue), such: *Candida albicans*, *Staphylococcus epidermidis* and some Gram-negative organisms.
- •: The substrates especially the magenta, may darken the color of the blood medium after the incubation that reduces the clarity of the lecture and makes the observing of hemolytic reactions difficult.
- •: The chromogenic substrates that give coloration to the purple, red and magenta colonies doesn't contrast appropriately with the red color of the medium.

US-A-5620865 discloses a medium which allows detection of *Enterococci* microbes in a liquified sample, within as little as 24 hours. The medium also allows quantifying the amount of *Enterococci* present in a sample and may be used in rapid screening methods. The medium contains an effective amount of vitamin, amino acid, trace element and salt ingredients operable to allow viability and reproduction of *Enterococci* in the presence of a nutrient indicator. The nutrient indicator is provided in an amount sufficient to allow a detectable characteristic signal to be produced in the medium by the growth of *Enterococci.* The medium further contains effective amounts of selective agents which are active to prevent or inhibit the growth of non-target (i.e., non-enterococcal) microorganisms. An exemplary medium for the detection of *Enterococci* in a sample is disclosed and includes (per liter) a biological buffer (e.g., about 5.0 to 7.0 grams N-tris[Hydroxy-methyl]methyl-3-amino-propanesulfonic acid, free acid (TAPS-free acid), and about 5.0 to 7.0 grams N-tris [Hydroxy-methyl]methyl-3-amino-propanesulfonic acid, sodium salt (TAPS-sodium) or about 4.0 to 5.0 grams HEPES free acid and about 7.0 to 9.0 grams HEPES sodium salt)); and sodium bicarbonate (e.g., about 1.5 to 2.5 grams). The document discloses as nutrient indicator 4-methylumbelliferyl-beta-D-glucopyranoside. Other nutrient indicators which may be used according to US-A-5620865 include the following: 5-bromo-4-chloro-3-indoxyl-beta-D-glucopyranoside, o-nitrophenyl-beta-D-glucopyranoside, p-nitrophenyl-beta-D-glucopyranoside, resofuran-beta-D-glucopyranoside, 6-bromo-2-naphthyl-D-glucopyranoside, Rose-beta-D-glucopyranoside, VQM-Glc (2-{2-[4-(D-glucopyranosyloxy)-3-methoxyphenyl]vinyl)1-methylquinolinium iodide, and VBzTM-Gluc(2-{2-[4-(-D-glucopyranosyloxy)3-methoxyphenyl]vinyl}-3-methylbenzothiazolium iodide.

FR-A-2728587 discloses a nonselective culture medium allowing the numeration and the identification of germs in a biological sample, notably of urine. The medium comprises:(a) nutrient elements allowing the growth of germs potentially responsible for urinary infections; (b) a beta -D-glucuronidase chromogenic substrate containing 5-bromo-6-chloro-3-indolyl beta -D-glucuronide (I) or a salt, and(c) a beta - glucosidase chromogenic substrate.

US-A-5464755 discloses methods and media for the isolation and presumptive identification of various bacteria. In particular, the organisms commonly associated with urinary tract infections are distinguished based on their colonial morphology and color. A method is disclosed for detecting the presence of bacterial organisms in a test sample suspected of containing bacteria. The used medium comprises: a chromogenic beta-glucuronidase substrate capable of forming a first color upon reacting with a beta-glucuronidase; and a chromogenic arylsulfatase substrate capable of forming a second color upon reacting with arylsulfatase. It mentions chromogenic beta-glucuronidase substrate such as 6-chloro-3-indolyl-beta-D-glucuronide, and 5-bromo-6-chloro-3-indolyl-beta-D-glucuronide. Further as chromogenic arylsulfatase substrate is mentioned indoxyl-3-sulfate. The medium may further contain pH indicators such as m-cresol purple, phenol red, thymol blue, bromthymol blue, bromcresol purple, xylenol blue, and cresol red. The medium may further contain coordinating compound such as manganese, copper and iron ions.

US-A-4259442 discloses a rapid identification of different species of *Streptococcus* by: culturing the bacteria for several hours under unusual conditions which induces the bacteria to create characteristic enzymes by which the bacteria can be identified such as pyrrolydonyl arylamidase, beta-glucuronidase, beta-glucosidase, N-acetyl-beta-glucosaminidase and phosphatase. The medium contains no more than about I g of glucose per liter of culture medium to produce a dense culture. The culture is distributed onto several supports containing different substrates which are capable of reacting with enzymes so produced by different species of *Streptococcus* and the culture is incubated to produce a distinctly colored or colorable reaction product. Streptococci A and D are identified by the enzymatic activity of pyrrolydonyl arylamidase, by hydrolytic splitting of pyrrolydonyl-beta-naphthylamide or pyrrolydonylnitroanilide. Streptococci C and G are idientified by the enzymatic activity of B glucuronidase, by hydrolytic splitting of naphthol-beta-D-glucuronic acid or methylumbelliferyl-beta-D-glucuronic acid. *Streptococcus* D is idientified by the enzymatic activity of N-acetyl-beta-glucosamidinase, by hydrolytic splitting of naphthyl-N-acetyl-beta-D-glucosamine or the corresponding nitrophenyl or methylumbelliferyl derivatives, such as nitrophenyl-N-acetyl-beta-D-glucosamine or methylumbelliferyl-N-acetyl-beta-D-glucosamine. *Streptococcus* D is identified by the enzymatic activity of beta-glucosidase, by hydrolytic splitting of 6-bromo-2-naphthyl-.beta.-D-glucopyranoside or the corresponding nitro-phenyl or methyl umbelliferyl derivatives, such as nitrophenyl-.beta.-D-glucopyranoside or methyl umbelliferyl-.beta.-D-glucopyranoside. Streptococci A, B, C and G are identified by the enzymatic activity of phosphatase, by hydrolytic splitting of 2-naphthylphosphate or nitro-phenylphosphate.

### Summary of the Invention

The objective of the present invention consists in providing a selective medium for the isolation and detection of species of *Streptococcus* genus, facilitating the differentiation of *Streptococcus* of clinical importance, such as: *Streptococcus agalactiae, Streptococcus pyogenes* and D group *Streptococcus (Enterococcus),* in early cultivation periods (2-24 hours) and with a high sensibility and analytic specificity.

The innovation of the proposed invention consists in a combination of nutrient bases, fundamentally derived of the proteins, specially selected in a total quantity from 15 to 58 g/l with a total content of nitrogen between 10 and 14%. Moreover it consists in a specially designed mixture of inhibitors of Gram-negative microorganisms in quantities from 0,55 to 1,63 g/l wherein said mixture consists of thallium acetate and nalidixic acid. Additionally it contains a mixture of chromogenic and/or fluorogenic substrates, which must reveal at least one specific enzyme of each species for revealing phosphatase, glucosidase and glucuronidase activity selected among p-nitrophenylphosphate disodium salt, 5-bromo-4-chloro-3-indolyl-βD-glucopyranoside, 4-methylumbelliferyl-βD-glucopyranoside, 6-chloro-3-indolyl-βD-glucopyranoside, 5-bromo-4-chloro-3-indolyl-βD-glucuronide cyclohexylammonium salt, 5-bromo-6-chloro-3-indolyl-βD-glucuronide cyclohexylammonium salt and 4-methylumbelliferyl-βD-glucuronide trihydrate, in quantities from 0,3 to 1,5 g/l and a mixture of compounds indicative of deaminas activity, in total quantities of 1 to 3 g/l, wherein said mixture consists of trivalent metal salts and aromatic amino acids.

The original mixture of nutrient bases is composed by extracts and hydrolysates of different origins, specifically selected among
- •: Hydrolyzate of bovine blood of 5 to 10 g/l
- •: Beef heart extract of 1 to 12 g/l
- •: Enzymatic hydrolysate of beef heart muscle of 1 to 5 g/l
- •: Enzymatic hydrolysate of milk proteins of 5 to 20 g/l
- •: Enzymatic Hydrolysate of Soja bean proteins of 2 to 15 g/l
- •: Enzymatic Hydrolysate of animal tisues of 1-5 g/l
- •: *Saccharomyces cerevisiae* yeast autolyzate or hydrolysate of 2 to 15 g/l

The never described peculiar mixture of inhibitors includes a combination of Thallium acetate from 0,55 to 1,6 g/l Nalidixic acid from 0,005 to 0,030g/l

Another singular feature of the invention consists in the employment of the mixture of chromogenic and fluorogenic substrates that reveal the phosphatase, glucosidase and glucuronidase activity to differentiate the diverse *Streptococcus* species. It contains p-nitrophenylphosphate disodium salt (PNP), from 0,2 to 0,8 g/l; x-glu (5-bromo-4-chloro-3-indolyl- beta-D-glucopyranoside, Mu-glu (4-methylumbelliferyl-beta -D-glucopyranoside), Salmon-glu (6-chloro-3-indolyl- beta -D-glucopyranoside) from 0,05 to 0,4 g/l and x-gluc (5-bromo-4-chloro-3-indolyl-beta - D-glucuronide, cyclohexylammonium salt), Magenta-Gluc (5-bromo-6-chloro-3-indolyl- beta -D-glucuronide, cyclohexylammonium salt) and MUG (4-methylumbelliferyl- beta -D-glucuronide trihydrate) from 0,05 to 0,2 g/l.

These chromogenic substances combine in a very original form to guarantee the appearance of colors or fluorescences different for each species to detect. Among them can be mentioned
a) PNP + x-glu + Magenta-gluc or MUG
b) PNP + Salmon-glu + x-gluc or MUG
c) PNP + MU-glu + Magenta gluc or x-gluc

Another singular aspect of the invention, consists in that are added several compounds, which indicate the deaminase activity in the medium, such as trivalent metals salts, preferably ferric ammonium citrate in quantities from 0,5 to 1,0 g/l and aromatic amino acids, preferably phenylalanine and tryptophan, in quantities from 1,0 to 2,0 g/l.

To the before mentioned composition from 10,0 to 14,0 g/l of agar is incorporated. The obtained medium allows observing the cultural characteristics of the target organisms.

The formulated medium presents the pH values from 7 to 7,4 and its concentration in water may be varied from 25 to 80 g/l.

The medium permits, for the first time, to isolate and detect exclusively different species of *Streptococcus,* fundamentally human pathogens, only by specific colony colors of certain genera and changes of the medium coloration.

For the first time is provided a selective medium that facilitates at the same time the differentiation of D group streptococci (*Enterococcus*) of the rest of them, and between the species of *Streptococcus* genus. For example, *Streptococcus agalactiae* and *Streptococcus pyogenes* that grow well while other Gram-positive and Gram-negative species are inhibited.

The advantages of the medium proposed in the following invention consist in that:
- •: For the first time the differentiation between the pathogenic and most of non- pathogenic species of *Streptococcus* is facilitated.
- •: The medium is highly selective for *Streptococcus,* inhibits most of Gram negative bacterial species, except *Proteus mirabilis* ATCC 7002 which growth is very inhibited in the medium, moreover this organism can be identified by change of the medium color to one different to Streptococci species. Also very few species of other inhibited Gram positive organisms may develop a minimal growth in the medium and they take a coloration that doesn't interfere with the abundant growth of the target microorganisms.
- •: The medium contains the complex and novel mixture of nutrient bases obtaind from dissimilar sources with various compositions, which provides the presence of amino acids, peptides, polypeptides, proteoses and peptones, and unexpectedly, they facilitate an abundant growth of the target species and allow the exclusion of labile substances such as hemin and blood.
- •: The fact of special relevance and novelty is the inclusion of the blood enzymatic hydrolyzate in the before mentioned mixture. It guarantees the presence in the medium of blood derived substances or its fractions that are necessary for the *Streptococcus* growth and avoid the identification hindering. On the other hand, the digested blood proteins don't affect stability of the medium.
- •: Due to the presence of a sufficient quantities of nutrients for the quick growth of streptococci in the medium, and the inhibitory substances that provides the absence of the other competitor organisms, the members of this genus can be developed faster and can be isolated and identified no later than after 6 to 24 hours of incubation.
- •: The sensitivity and specificity values are high for the reason that the streptococci cannot be confused with Gram negative bacteria, such as: *Shigella* and *Escherichia coli,* because these microorganism don't grow in the medium. Also a few Gram-positive species that are able to grow in the medium (*Staphylococcus xylosus* and *Staphylococcus saprophyticus*) have different cultural characteristics and are almost totally inhibited so cannot be confused with the rest of the microorganisms. If some strain presents the growth characteristics similar to *Streptococcus,* it can be differed by direct catalase test in the plate.
- •: In the present invention are not used neither highly toxic substances nor environment pollutants. For this reason, the medium can be prepared easily such by industrial scale as in the laboratory, under appropriate and specific conditions of the activity.
- •: The effectiveness of the inhibitors mentioned in the formulation avoids the use of labile antibiotic, so the medium preparation is simple and don't exist identification mistakes due to phenomena of resistance to the antimicrobial agents.
- •: The inhibitor combination and concentration in the medium and its ratio regarding to the nutrients makes possible that none *Streptococcus* species aren't inhibited in the medium. This fact makes the medium very suitable, as never before, for the specific diagnosis of this genus.
- •: The faster diagnosis can be obtained because when the target streptococci are detected and differed appropriately, with high sensitivity and specificity values, it is not necessary to carry out additional biochemical tests.
- •: The preparation of this medium is extremaly simple because, in some cases, it can not be autoclaved, and in other, only one supplement must be added.
- •: The stability of the prepared medium is large, because it contains inhibitors of most of environmental pollutants and doesn't contain labile substances (as the hemin, blood and antibiotics).
- •: When the identification of the target bacteria is only carried out by the cultural characteristics (medium color and colonies), it excludes the necessity to observe detailedly morphological characteristics of the colonies, facilitating the interpretation of the results for a trained but not specialized personnel.
- •: The use of more than one enzymatic marker for the differentiation of the diverse species of the *Streptococcus* genus increases the diagnostic specificity and sensitivity.
- •: The formulation reported in the application facilitates to prepare the medium to different concentrations for multiple presentations (for example, to simple concentration in gelled medium, to double concentration in the membrane filtration techniques, or in broth form, and even to a higher concentration for miniaturized systems).
- •: With this medium, it is possible to substitute the employment of different traditional or chromogenic media used in the identification of different species within *Streptococcus* genus.
- •: The proposed formulation facilitates its employment in the screening of *Streptococcus* in clinical or veterinary samples, in waters and other elements of the environment as well as in foods.
- •: The isolation and simultaneous identification is achieved in a single step, with the consequent reduction of time, of resources and personal.

### Detailed description of the invention

For the definition of the composition of the medium, the components used in the preparation were preferably solids. The description of the steps that must be done for the preparation of the medium is presented below.

The mixture of nutrient bases which total nitrogen content is between 10 and 14%, is sifted to achieve uniformity in the size of the particles.

The quantity of these ingredients of proteic origin is varied from 15 to 58 g/l. Specifically the quantity ranges are described below.
- •: Bovine blood hydrolysate from 5 to 10 g/l,
- •: Beef heart extract from 1 to 12 g/l,
- •: Beef heart muscle enzymatic hydrolysate from 1 to 5 g/l,
- •: Milk protein enzymatic hydrolysate from 5 to 20 g/l,
- •: Soja bean protein enzymatic hydrolysate from 2 to 15 g/l,
- •: Animal tissue enzymatic hydrolysate from 1 to 5 g/l,
- •: *Saccharomyces cerevisiae* yeast autolyzate or hydrolysate from 2 to 15 g/l.

The selective agents are selected among: thallum acetate from 0,55 to 1,6 g/l and nalidixic acid from 0,05 to 0,030g/l. By the same way they are sifted to stay ready for mixing with rest of the compounds.

The chromogenic and fluorogenic compound to detect the glucosidase, phosphatase and glucuronidase activity in quantities from 0,3 to 1,5 g/l, are premixtured. Furthermore, they may be milled and sifted previously to the homogenization. If the mixing procedure is efficient weighing the premixture quantity equal to the sum of the quantities of each component in the formulation, an appropriate distribution of each one must be achieved.

According to the medium purpose, chromogenic and fluorogenic substrates may be selected among
- •: p-nitrophenylphosphate disodium salt (0,2 to 0,8 g/l);
- •: x-glu (5-bromo-4-chloro-3-indolyl- beta D-glucopyranoside), Mu-glu (4- methylumbelliferyl- beta D-glucopyranoside), Salmon-glu (6-chloro-3-indolyl - beta D-glucopyranoside) (0,05 to 0,4 g/l);
- •: x-gluc (5-bromo-4-chloro-3-indolyl- beta D-glucuronide cyclohexylammonium salt), Magenta-gluc (5-bromo-6-chloro-3-indolyl- beta D-glucuronide cyclohexylamonium salt) and MUG (4-methylumbelliferyl- beta D-glucuronide trihydrate)(0,05 to 0,2 g/l).

The conformation of the mixture of chromogenic and fluorogenic substances that may be degradable by at least one enzyme of each species was carried out. The combinations that guarantee the appearance of colors or different fluorescences for each species are described next:
a) PNP + x-glu + Magenta-gluc or MUG
b) PNP + Salmon-glu + x-gluc or MUG
c) PNP + Mu-glu + Magenta-gluc or x-gluc

The gelling agent, preferably agar, with gel strength between 400 and 700 g/cm2 , is used in a range of 10,0 to 14,0 g/l, specifically for the solid medium. Before be added to the medium it must be dried and sifted.

All the before mentioned compounds, as well as the premixture, are united and homogenized until achieving an uniform mix with a pH value between 7,0 and 7,4. The mixture is distributed in tightly closed flasks, which are protected from the light and maintained at environmental temperature.

The powder is suspended in destilled or deionized water in quantities from 30 to 80 g/l.

The composition can be made at laboratory scale, weighing the ingredients separately inside a recipient and maintaining the before mentioned proportions. Later little by little is added the water on the powder mixture, until achieving complete dissolution. Mix thoroughly the suspension and allow to rest at least 10 minutes.

If solid medium must be prepared, this may be either sterilized or not before the addition of the p-nitrophenylphosphate disodium salt. If cautions have been taken in the preparation, it is enough the heating until to boil, because the medium is highly inhibitory for environmental microorganisms that could spoil it. Otherwise, the medium must be sterilized for 10 minutes at 115 DEG C and then, after it is cooled to 45-50 DEG C, sterile filtered PNP solution is added. In case of the liquid medium, it is always sterilized in autoclave and then the sterile supplement is added. When the broth is prepared, it must be distributed in the final containers.

In dependence of the medium consistency, solid or liquid, different inoculation methods may be used.

Once inoculated, they are incubated at 30 to 37 DEG C for at least from 2 to 6 hours in case of the liquid medium and from 18 to 24 hours in case of the solid medium.

The reading of the results is carried out in the case of the solid medium, observing the color of the colonies isolated in the surface of the medium. For the liquid medium only is observed the change of medium coloration and the fluorescence. So the D group streptococci can be differed from the rest of the species of this genus. The staphylococci, generally, are not developed in the medium or may be very inhibited and observed as colorless small colonies (*Staphylococcus saprophyticus*) or with the colony colors different from the streptococci and so don't interfere in the identification of the target organisms.

The enterococci is observed of rose or blue color or with fluorescens according to the combination of the used substrates, while in dependence of the used combination the streptococci is observed yellow, yellow greenish, green, magenta, blue or fluorescent, whenever is used a color substrate different to the employed to reveal the enterococci presence.

### EXAMPLES OF REALIZATION:

### Example No. 1

In this experiment, the effects of a series of nutrient base mixtures were studied as nutritional components in the formulation. In the experiments 3 variants were designed whose composition appears in the table 1. The ingredients were reconstituted in 100 mL of water and the composition was prepared according to the detailed description of the present invention.

The evaluation of the growth promoting capacity was carried out with collection strains in comparison with reference medium (Selective Broth of Streptococci Enrichment, Merck, lot: 86444327).

In each tube was inoculated 0,1 mL of standardized solution at 50% of transmittance of each microorganism.

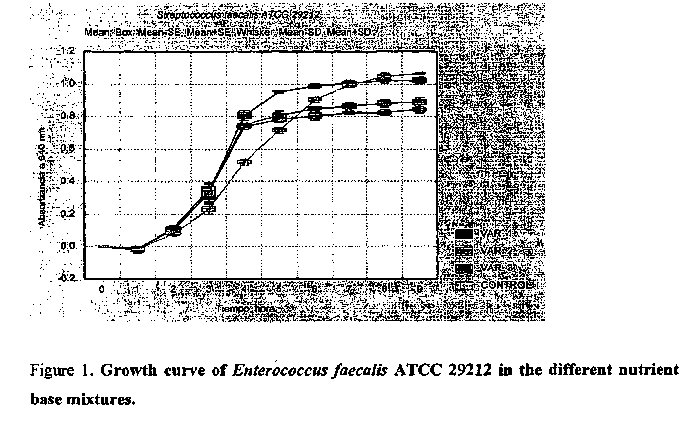

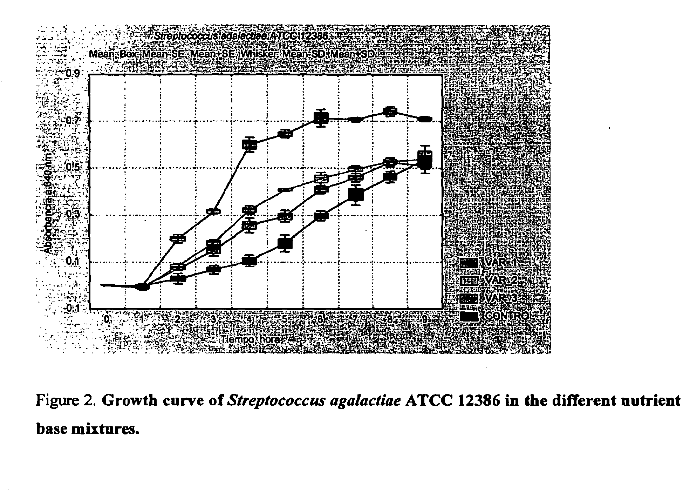

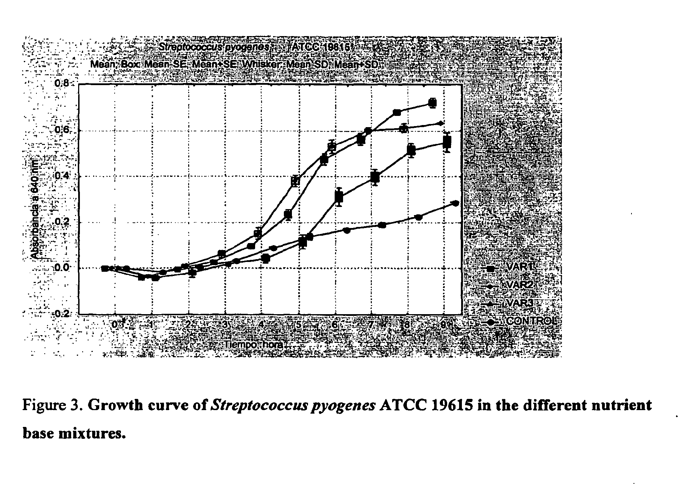

The growth curves are shown in the figures 1, 2 and 3.

The results of the promotion of streptocococi growth were highly satisfactory. In the case of *Streptococcus agalactiae* it was observed that the 3 characteristic compositions of the present invention promoted the cell development during the first 8 hours with more intensity with regard to the control medium and even the variant I promoted the growth in a very superior form until the 9 hours.

In the case of *Streptococcus faecalis* a larger acceleration of the growth was observed in the first 4 hours, in the experimental variants, with regard to the control, and in the case of *Streptococcus pyogenes*, starting from the 6 hours the growth was significantly superior in the new proposed compositions.

**Table 1**

| **Composition of nutrient bases of the different experimental variants** | | | |
|---|---|---|---|
| **Component** | **Composition (g/100 mL)** | | |
| | **V1** | **V2** | **V3** |
| *Saccharomyces cerevisiae* autolyzate or yeast hydrolyzate | 0,32 | 0,32 | 0,32 |
| Beef heart extract | 0,24 | 0,24 | 0,24 |
| Milk protein enzymatic hydrolyzate | 0,97 | 0,97 | 0,97 |
| Soja bean protein enzymatic hydrolyzate | 0,32 | - | - |
| Beef heart muscle enzymatic hydrolyzate | - | - | 0,22 |
| Animal tissue enzymatic hydrolyzate | - | 0,46 | - |
| **Total of the mixture** | 1,85 | 1,99 | 1,75 |
| **Total Nitrogen of the mixture** | 13,30 | 12,36 | 12,31 |

### Example No. 2

The composition was prepared with the ingredients according to example 1, but weighed separately into a Erlenmeyer flask. As growth promoting agents and enzymatic markers the following ingredients were used:

**Table 2**

| **Component** | **g/100 mL** |
|---|---|
| Milk protein enzymatic hydrolyzate | 0,97 |
| *Saccharomyces cerevisiae* autolyzate or yeast hydrolyzate | 0,32 |
| Beef heart extract | 0,24 |
| Soja bean protein enzymatic hydrolyzate | 0,32 |
| p-nitrophenylphosphate disodium salt | 0,04 |
| x-glu (5-bromo-4-chloro-3-indolyl-βD-glucopyranoside) | 0,02 |

In the formulation agar was not used. To the mixture of solid ingredients 100 mL of desionized water was added and the composition was prepared as disclosed in the detailed description.

Inoculations were performed with collection strains of *Streptococcus pyogenes* ATCC 19615, *Streptococcus agalactiae* ATCC 12386, *Enterococcus faecalis* ATCC 29212, *Enterococcus faecalis* ATCC 19433, *Enterococcus faecium* ATCC 6056. These strains were inoculated in a direct form with a standardized solution at 50% of transmittance in each of the tubes.

It was shown that after 18 hours the medium appears blue-greenish in the case of *Enterococcus faecalis* ATCC 29212 and 19433. The strain of *Enterococcus faecium* showed dark blue coloration, *Streptococcus agalactiae* appeared green and for *Streptococcus pyogenes* a yellow-greenish color was observed.

The results show the speed of the microorganism response in the time and the possibility of differentiating the enterococci (group D) from the rest of the streptococci because they exhibit a coloration from green-blue to blue and the streptococi, such as *Streptococcus pyogenes* and *Streptococcus agalactiae,* show a greenish yellow coloration.

To check the certainty of the selection of the x-glu chromogenic substrate a medium with similar composition to the one described above was prepared, with the difference that esculin was used. The used quantity was of 0,1 g/100mL, iron salts were also added in a concentration of 0,05 g/100mL.

These ingredients were weighed separately into an Erlenmeyer flask, thereafter 100 mL of deionized water was added and the composition was prepared.

Inoculations were performed with collection strains of *Streptococcus pyogenes* ATCC 19615, *Streptococcus agalactiae* ATCC 12386, *Enterococcus faecalis* ATCC 29212 and some isolated strains of clinical samples (4 enterococci and 4 hemolytic streptococci). These strains were inoculated in a direct form with a standardized solution at 50% of transmitance in each of the tubes.

The obtained results for both strains, *Enterococcus faecium* and *Enterococcus faecalis* was a change of the medium color to black, which masks the other enzymatic reactions to which some species can be positive.

Both *Streptococus pyogenes* and *Streptococcus agalactiae* strains appear of color olive green. The 4 isolated strains of enterococi gave black color, the same aswer exhibit by 3 hemolytic streptococci. Only a hemolytic microorganism was observed of olive green color the same as *Streptococus pyogenes* and *Streptococcus agalactiae.*

The obtained results were seen early, but the blackish coloration resulting from esculin hydrolysis limits the identification, which prevents the reading of the rest of the reactions.

As a conclusion the experiment allowed to evaluate the possibility to include x-glu as ingredient in the formulation of the medium, and not the use of esculin.

### Example No. 3

The medium composition was prepared according to Example 2, with the difference that the concentration of the nutrient components and the enzymatic markers was increased. They were weighed separately into an Erlenmeyer flask in the following quantities:

**Table 3**

| **COMPONENT** | **g/100mL** |
|---|---|
| Milk protein enzymatic hydrolyzate | 1,94 |
| *Saccharomyces cerevisiae* autolyzate or yeast hydrolyzate | 0,64 |
| Beef heart extract | 0,48 |
| Soja bean protein enzymatic hydrolyzate | 0,64 |
| p-nitrophenylphosphate disodium salt | 0,08 |
| x-glu (5-bromo-4-chtoro-3-indotyl-βD-glucopyranoside) | 0,04 |

Collection strains were inoculated, such as: *Streptococcus pyogenes* ATCC 19615, *Streptococcus agalactiae* ATCC 12386, *Enterococcus faecalis* ATCC 29212, *Enterococcus faecalis* ATCC 19433, *Enterococcus faecium* ATCC 6056 and some isolated strains: *Enterococcus avium* (isolated). These strains were inoculated in a direct form in the wells of a polypropylene module (Module 250 pp, NUNC, lot: 046247) adding 0,1 mL of a standardized solution at 3,0 McFarland in 0,1 mL of medium.

After 2 hours changes in medium coloration were observed to blue-greenish for *Enterococcus faecalis* ATCC 29212, blue for *Enterococcus faecalis* ATCC 19433, *Enterococcus avium* appears yellow-greenish, *Enterococcus faecium* gave clear blue color, *Streptococcus pyogenes* and *Streptococcus agalactiae* showed an intense yellow color.

The answer stayed in the time (until the 6 hours). The results demonstrated the speed in the answer to the enzymatic reactions, and the possibility of developing a rapid test for the identification of species of *Streptococcus* genus.

### Example No. 4

The composition of the ingredients was prepared according to the example 2, with the addition of an gelling agent (agar) in a concentration of 6,5 g/500 mL. The components were weighed separately into an Erlenmeyer flask and to the mixture of solid ingredients 500 mL of deionized water was added. Thereafter, the preparation was prepared according to the example 1.

Collection strains were inoculated, such as: *Streptococcus pyogenes* ATCC 19615, *Enterococcus faecalis* ATCC 29212, *Enterococcus faecalis* ATCC 19433, *Enterococcus faecium* ATCC 6056.

They were inoculated by spreading the microorganism onto the surface of the medium, until achieving isolated colonies.

After 24 hours in the case of *Streptococcus pyogenes* a green-clear coloration of the colonies with change of the medium to yellow was achieved. The strains of *Enterococcus faecalis* ATCC 29212 and 19433 showed similar cultural characteristics, showing a blue coloration surrounded by a small blue halo. As for *Enterococcus faecium,* the observed colonies were smaller than the rest of the enterococci, and the colonies were of blue color surrounded by a small blue halo.

### Example No. 5

The composition of the medium was prepared by separately weighing the ingredients into an Erlenmeyer flask according to the example 4, with the difference that the enzymatic markers are in a smaller concentration (0,2 g/l p-nitrophenylphosphate disodium salt and 0,1g/l of 5-bromo-4-chloro-3-indolyl- beta D-glucopyranoside). The preparation method was the one described in the example 1.

They were inoculated by spreading the microorganism onto the surface of the medium, until achieving isolated colonies, collection strains of: *Streptococcus pyogenes* ATCC 19615, *Enterococcus faecalis* ATCC 29212, *Enterococcus faecium* ATCC 6056, *Staphylococcus aureus* ATCC 25923 and an isolated strain of *Enterococcus avium.*

The reading carried out after 24 hours of evaluating the composition with the collection strains was observed as shown in the table 4.

**Table 4**

| **Characteristics of growth and of the microorganism colonies in the solid medium.** | |
|---|---|
| **Strain** | **Characteristic of the colonies and of the medium** |
| *Streptococcus pyogenes* ATCC 19615 | Greenish colonies with change of medium color to yellow |
| *Enterococcus faecalis* ATCC 29212 | Blue colonies |
| *Enterococcus faecium* ATCC 6056 | Blue colonies |
| *Staphylococcus aureus* ATCC 25923 | White colonies with change of medium color to yellow |
| *Enterococcus avium* (isolated) | Blue colonies |

### Example No. 6

The composition was prepared with the ingredients according to Example 2, with the difference that the chromogenic substrate x-glu (5-bromo-4-chloro-3-indolyl- beta D-glucopyranoside) was substituted for Mu-glu (4-methylumbelliferyl- beta D-glucopyranoside) in a concentration of 0,05 g/l. The preparation method went similar to the example 2.

In the evaluation collection strains were used of: *Enterococcus faecalis* ATCC *29212, Enterococcus faecium* ATCC 6056, *Streptococcus lactis* ATCC 11454 and an isolated strain of *Enterococcus avium.* This strains were inoculated in a direct method in the wells of a polypropylene module (Module 250 pp, NUNC, lot: 046247) adding 0,1 mL of a standardized solution at 2,0 McFarland in 0,1 mL of medium.

After 2 hours, it was possible to detect the growth of the test microorganisms and to detect the blue blue fluorescence color of *Enterococcus faecalis* and *Enterococcus faecium* under ultraviolet light (365 nm).

The reading at 6 hours showed that all the inoculated strains possessed glucosidase activity, because they responded with a blue fluorescence color when lit with ultraviolet light.

It was demonstrated that it is possible to use fluorogenic substrate Mu-glu (4-methylumbelliferyl - beta D-glucopiranoside), because the detection of streptococci (*Enterococcus*) was rapid.

### Example No. 7

The medium composition was prepared by separately weighing the ingredients into an Erlenmeyer flask. Two inhibitors mixtures were tested in the solid medium. Hereafter the composition of growth promoter agents, enzymatic markers and used inhibitors is described:

**Table 5**

| **COMPONENT** | **g/200 mL** | |
|---|---|---|
| | V1 | V2 |
| *Saccharomyces cerevisiae* autolyzate or yeast hydrolyzate | 1,944 | 1,944 |
| Beef heart extract | 0,648 | 0,648 |
| Milk protein enzymatic hydrolyzate | 0,486 | 0,486 |
| Soja bean protein enzymatic hydrolyzate | 0,648 | 0,648 |
| p-nitrophenylphosphate disodium salt | 0,04 | 0,04 |
| Salmon - glu (6-chloro-3-indolyl - OD-glucopyranoside) | 0,02 | 0,02 |
| x-gluc (5-bromo-4-chloro-3-indolyl-βD-glucuronide cyclohexylammonium salt) | 0,02 | 0,02 |
| Talium acetate | 0,12 | 0,12 |
| Nalidixic acid | 0,003 | 0,002 |
| Agar | 2,6 | 2,6 |

Next the preparation was prepared according to the example 1.

The used inoculation method was spreading the microorganism onto the surface of the medium, until achieving isolated colonies. In the evaluation collection strains were used of: *Proteus mirabilis* ATCC 7002, *Proteus mirabilis* ATCC 12433, *Staphylococcus epidermidis* ATCC 12228, *Staphylococcus aureus* ATCC 25923, *Staphylococcus xylosus* ATCC 29971, *Staphylococcus saprophyticus* ATCC 15305, *Enterococcus faecium* ATCC 6056, *Streptococcus agalactiae* ATCC 12386, *Streptococcus faecalis* ATCC 29212 and the isolated strain of *Enterococcus avium.*

After 24 hours the reading was carried out and the results of the behavior of the microorganisms in the medium are shown in the table 6. It should be noted that the results obtained in both variants (V1 and V2) were similar.

**Table 6**

| **Characteristic of the medium and colonies** | | |
|---|---|---|
| **Strain** | **Growth** | **Color of the medium and colonies** |
| *Sfreptococcus aga*/*actiae ATCC 12386* | + | Green-blue colonies with change of medium coloration to yellow |
| *Enterococcus faecalis* ATCC 29212 | + | Rosy colonies |
| *Enterococcus faecium* ATCC 6056 | + | Rosy colonies |
| *Enterococcus avium* (isolated) | + | Rosy colonies |
| *Staphylococus saprophylicus* ATCC 15305 | poor | White colonies |
| *Staphylococcus xylosus* ATCC 29971 | + | Blue colonies |
| *Staphylococcus aureus* ATCC 25923 | + | White colonies |
| *Proteus mirabilis* ATCC 7002 | - | - |
| *Proteus mirabilis* ATCC 12433 | - | - |
| Legend: | | |
| +: good growth | | |
| -: Inhibited growth | | |

The strains of *Proteus mirabilis* stayed inhibited, evidencing the inhibitory power of the medium for Gram negative organisms.

Some strains of *Staphylococcus* were developed in the medium, however they took colorations different to the streptococci, but it was also carried out the rapid test of catalase, adding a drop of the reagent in the medium, being achieved the complete differentiation of both genera.

### Example No. 8

The composition of the ingredients was prepared according to example 7, the components were weighed separately into an Erlenmeyer flask with the difference that x-gluc was substituted (5-bromo-4-chloro-3-indolyl- beta D-glucuronide cyclohexylammonium salt) for Magenta-gluc (5-bromo-6-chloro-3-indolyl- beta D-glucuronide cyclohexylammonium salt).

The preparation method went similar to the example 7.

Collection strains of *Proteus mirabilis* ATCC 7002, *Staphylococcus epidermidis* ATCC 12228, *Staphylococcus aureus* ATCC 25923, *Staphylococcus xylosus* ATCC 29971, *Enterococcus faecium* ATCC 6056, *Streptococcus agalactiae* ATCC 12386, *Streptococcus pyogenes* ATCC 19615, *Enterococcus faecalis* ATCC 29212, *Staphylococcus saprophyticus* ATCC 15305 and the isolated strain of *Enterococcus avium* were inoculated.

The results of the behavior of the microorganisms in the medium are shown in the Table 7.

Table 7: The characteristics of the media and the colonies

**Table 7**

| **Characteristic of the medium and colonies** | | |
|---|---|---|
| **Strain** | **Growth** | **Color of the medium and colonies** |
| *Streptococcus pyogenes* ATCC 19615 | + | White colonies with change of medium coloration to yellow |
| *Streptococcus agalactiae* ATCC 12386 | + | Magenta colonies with slight change of medium coloration to yellow |
| *Enterococcus faecalis* ATCC 29212 | + | Rosy colonies |
| *Enterococcus faeclum* ATCC 6056 | + | Rosy colonies |
| *Enterococcus avium* (isolated) | + | Rosy colonies |
| *Staphylococus saprophyticus* ATCC 15305 | - | - |
| *Staphylococcus xylosus* ATCC 29971 | + | Magenta colonies |
| *Staphylococcus aureus* ATCC 25923 | + | White colonies |
| *Proteus mirabilis* ATCC 7002 | - | - |
| Legend: | | |
| +: good growth | | |
| -: inhibited growth | | |

An early and differentiated growth of the species of *Streptococcus* was observed for the different colorations developed in the medium.

Some strains of *Staphylococcus* were developed in the medium and they showed colorations different to streptococci, although *Staphylococcus xylosus* appears of a similar color to *Streptococcus agalactiae,* but it is possible to differentiate because it doesn't change the medium color to yellow. Nevertheless a rapid test of catalase was carried out, by adding a drop of the reagent in the medium, and by liberation of the products H₂ and O₂ (formation of bubbles) the identification of *Staphylococcus* genus was achieved.

### Example No. 9

The composition was prepared according to example 8, with the difference that X-gluc was substituted by the fluorogenic substrate MUG, in a concentration of 0,05 g/l. To the mixture of solid ingredients deionized water was added and the composition was prepared until their gelling process as it is described in example 1. Collection strains of *Proteus mirabilis* ATCC 7002, *Staphylococcus aureus* ATCC 25923, *Staphylococcus xylosus* ATCC 29971, *Enterococcus faecium* ATCC 6056, *Streptococcus agalactiae* ATCC 12386, *Streptococcus pyogenes* ATCC 19615, *Streptococcus faecalis* ATCC 29212, *Staphylococcus saprophyticus* ATCC 15305 and the isolated strain of: *Enterococcus avium*were inoculated by spreading the microorganism onto the surface of the medium. The reading was carried out after 24 hours, according to the table 8.

**Table 8**

| **Characteristics of the growth and the colonies at the 24 hours of Incubation** | | | |
|---|---|---|---|
| **Strain** | **Growth** | **Characteristic of the colonies and medium** | **Fluorescence** |
| *Streptococcus agalactiae* ATCC 12386 | + | White colonies with change of medium color to yellow | + |
| *Streptococcus pyogenes* ATCC 19615 | + | White colonies with change of medium color to yellow | - |
| *Enterococcus faecalis* ATCC 29212 | + | Rosy colonies with change of medium color to yellow | - |
| *Enterococcus faecium* ATCC 6056 | + | Rosy colonles | - |
| *Enterococcus avium* (aislado) | + | Rosy colonies | - |
| *Staphylococcus xylosus* ATCC 29971 | ± | White colonies | - |
| *Staphylococcus aureus* ATCC 25923 | ± | White colonies | - |
| *Staphylococcus saprophyticus* ATCC 15305 | - | - | - |
| *Proteus mirabilis* ATCC 7002 | - | - | - |
| Legend: +: good growth and positive fluorescence | | | |
| (: scarce growth | | | |
| -: inhibited growth | | | |

### Example No. 10

The medium composition was prepared by separately weighing the ingredients into an Erlenmeyer flask. The medium was prepared with the same proportion of nutrient bases as example 7, except that bovine blood enzymatic hydrolyzate was added in quantities of 1,911 g/300 mL. Two different enzymatic markers were also tested in concentration of 1,0 g/l with the addition of 0,5 g/l ferric ammonium citrate and two different concentrations of nalidixic acid 0,015 g/l and 0,010 g/l. The composition of enzymatic markers and used inhibitors is described below:

**Table 9**

| **COMPONENT** | **g/300 mL** | **g/300 mL** |
|---|---|---|
| | **V1** | **V2** |
| P - Nitrophenylphosphate disodium salt | 0,06 | 0,06 |
| Salmon - glu (6-chloro-3-indolyl-βD- glucopyranoside) | 0,0225 | 0,0225 |
| x-gluc (5-bromo-4-chloro-3-Indolyl-βD-glucuronide cyclohexylammonium salt) | 0,0225 | 0,0225 |
| Talium acetate | 0,18 | 0,18 |
| Nalidixic acid | 0,0045 | 0,003 |
| Tryptophan | 0,3 | - |
| Phenylalanine | - | 0,3 |
| Ferric ammonium citrate | 0,15 | 0,15 |
| Agar | 3,9 | 3,9 |

The preparation was according to the example 1.

The used inoculation method was: spreading the microorganism onto the surface of the medium and in the case of the organisms used as positive controls it was also inoculated by dilutions (10⁻⁴ and 10⁻⁵). In the evaluation collection strains were used of: *Proteus mirabilis* ATCC 7002, *Proteus mirabilis* ATCC 12433, *Citrobacter freundii* ATCC 8090, *Citrobacter freundii* ATCC 10625, *Enterococcus faecium* ATCC 6056, *Streptococcus agalactiae* ATCC 12386, *Enterococcus faecalis* ATCC 29212 and the isolated strain of *Enterococcus avium.*

The results of the behavior of the microorganisms in the medium are shown in the table 10.

**Table 10**

| **Evaluation of the behavior of the different microorganisms (incubation 35 °C for 24 h).** | | | |
|---|---|---|---|
| **Strain** | **Media** | **Characteristic and color of the isolated colonles** | **Promotion of the growth** |
| | | | UFC/mL |
| *Streptococcus agalactiae* ATCC 12386 | Experim. | Green-blue colonies with medium yellow | 365 |
| | ATS | White colonies | 330 |
| *Enterococcus faecalis* ATCC 29212 | Experim. | Rosy colonies | 600 |
| | ATS | White colonies | 445 |
| *Enterococcus taeclum* ATCC 6056 | Experim. | Rosy colonies | 310 |
| | ATS | White colonies | 65 |
| *Enterococcus avium* (Isolated) | Experim | Rosy colonies | 240 |
| | ATS | White colonies | 110 |
| *Citrobacter froundii* ATCC 8090 | Experim. | Inhibited | - |
| | ATS | Whitecolonies | |
| *Citrobacter freundil* ATCC 10625 | Experim. | Inhibited | - |
| | ATS | White colonies | |
| *Proteus mirabilis* ATCC 7002 | Experlm. | Inhibited | - |
| | ATS | White colonies | |
| *Proteus mirabilis* ATCC 12433 | Experim. | Inhibited | - |
| | ATS | White colonies | |
| Experim: Composition of the present invention | | | |
| ATS: AGAR TRIPTONE SOJA | | | |
| UFC/mL: Units Forming Colony per each mL of dilution 10⁻⁵ | | | |

In the medium the growth of *Streptocococcus agalactiae, Enterococcus faecalis, Enterocccus faecium* and *Enterococcus avium* to high dilutions is observed, and within the *Streptococcus* genus differentiated growth of the different species is demonstrated.

Strains of Gram negative microorganisms were inoculated, using traditional Agar Triptone Soja medium as reference. When carrying out the comparison with the control medium the inhibitory power of the formulation of this invention was proven when observing the total inhibition of the *Citrobacter* and *Proteus* genera.

### Brief Description of the Figures:

Figure 1: Growth curve of *Enterococcus faecalis* ATCC 29212 in the different nutrient base mixtures.
Figure 2: Growth curve of *Streptococcus agalactiae* ATCC 12386 in the different nutrient base mixtures.
Figure 3: Growth curve of *Streptococcus pyogenes* ATCC 19615 in the different nutrient base mixtures.

## Claims

1. Selective culture medium for the isolation and early detection of species of the Streptococcus genus, **characterized** to contain a mixture of nutrient bases of proteic origin in a total quantity of 15 to 58 g/l with a content of total nitrogen from 10 to 14% selected among bovine blood hydrolyzate, beef heart extract, beef heart muscle enzymatic hydrolyzate, milk protein enzymatic hydrolyzate, soja bean protein enzymatic hydrolyzate, animal tissue enzymatic hydrolyzate, Saccharomyces cerevisiae yeast autolyzate or hydrolyzate; a mixture of inhibitors of Gram-negative microorganisms in a total quantity of 0,55 to 1,63 /1, wherein said mixture consist s of thallium acetate and nalidixic acid; a mixture of degradable chromogenic and fluorogenic substances in a total quantity of 0.3 to 1.5 g/l for revealing phosphatase, glucosidase and glucuronidase activity selected among p-nitrophenylphosphate disodium salt, 5-bromo-4-chloro-3-indolyl-βD-glucopyranoside, 4-methylumbelliferyl-βD-glucopyranoside, 6-chloro-3-indolyl-βD-glucopyranoside, 5-bromo-4-chloro-3-indolyl-βD-glucuronide cyclohexylammonium salt, 5-bromo-6-chloro-3-indolyl-βD-glucuronide cyclohexylammonium salt and 4-methylumbelliferyl-βD-glucuronide trihydrate and a mixture of indicative compounds of deaminase activity, in a total quantity of 1 to 3 g/1, wherein said mixture consists of trivalent metal salts and aromatic amino acids.

2. Culture medium according to claim 1, **characterized** to contain nutrient bases of proteic origin in the following quantities within the medium:
• Bovine blood hydrolyzate of 5-10 g/l
• Beef heart extract of 1 - 12 g/1
• Beef heart muscle enzymatic hydrolyzate of 1-5 g/l
• Milk protein enzymatic hydrolyzate of 5-20 g/l
• Soja bean protein enzymatic hydrolyzate of 2-15 g/l
• Animal tissue enzymatic hydrolyzate of 1-5 g/l
• Saccharomyces cerevisiae yeast autolyzate or hydrolyzate 2 - 15 g/l.

3. Culture medium according to claim 1, wherein the inhibitors of Gram negative microorganisms are present in the following quantities within the medium:
• Thallium acetate of Q55 to 1,6 g/1
• Nalidixic acid of 0,005 to 0,030 g/1.

4. Culture medium according to claim 1, **characterized** to contain a mixture of degradable chromogenic and fluorogenic substances for at least one of the organisms to identify, selected in the following quantities within the medium:
• p-nitrophenylphosphate disodium salt (PNP) of 0,2 to 0,8 g/l
• 5-bromo-4-chloro-3-indolyl-βD-glucopyranoside (x-glu), 4-Methylumbelliferyl-BD-glucopyranoside (Mu-glul), 6-chloro-3-indolyl -BD-glucopyranoside (Salmon-glu) of 0,05 to 0,4 g/l.
• 5-bromo-4-chloro-3-indolyl-βD-glucuronide cyclohexylammonium salt (x-gluc), 5-bromo-6-chloro-3-indolyl-βD-glucuronide cyclohexylammonium salt (Magenta-Gluc) and 4-Methylumbelliferyl-βD-glucuronide trihydrate (MUG) of 0,05 to 0, 2 g/1.

5. Culture medium according to claim 1, **characterized** to contain a mixture of indicative compounds of deaminase activity in the following quantities within the medium:
• trivalent metal salts, preferably ferric ammonium citrate in quantities of 0,5 to 1,0 g/l
• aromatic amino acids, preferably tryptophan and phenylalanine in quantities of 1 to 2 g/1.

6. Culture medium according to claim 1, **characterized by** a mixture of degradable chromogenic and fluorogenic substances for revealing phosphatase, glucosidase and glucuronidase activity, in such combinations that guarantee the appearance of colors or different fluorescence for each species, selected from the group consisting of
a) PNP + x-glu + Magenta-gluc or MUG
b) PNP + Salmon-glu + x-gluc or MUG
c) PNP + Mu-glu + Magenta-gluc or x-gluc.

7. Culture medium according to claim 1, **characterized** to also contain agar with a gel strength between 400 and 700 g/cm² in quantities of 10 and 14 g/1 for the preparation of solid medium.

8. Culture medium according to the claim 1, wherein the pH value is from 7 to 7,4.

9. Culture medium according to claim 1, wherein its concentration in water varies from 25 to 80 g/1.

## Patentansprüche

1. Selektives Kulturmedium für die Isolierung und frühe Detektion von Spezies der Gattung Streptococcus, **dadurch gekennzeichnet, dass** es ein Gemisch von Nährstoffgrundlagen mit Proteinherkunft in einer Gesamtmenge von 15 bis 58 g/l mit einem Gehalt an Gesamtstickstoff von 10 bis 14%, ausgewählt aus Rinderbluthydrolysat, Rinderherzextrakt, enzymatischem Hydrolysat von Rinderherzmuskel, enzymatischem Hydrolysat von Milchprotein, enzymatischem Hydrolysat von Sojabohnenprotein, enzymatischem Hydrolysat von Tiergewebe, Saccharomyces cerevisiae-Hefe-Autolysat oder -Hydrolysat; ein Gemisch von Inhibitoren von Gram-negativen Mikroorganismen in einer Gesamtmenge von 0,55 bis 1,63 g/l, wobei das Gemisch aus Thalliumacetat und Nalidixinsäure besteht; ein Gemisch von abbaubaren chromogenen und fluorogenen Substanzen in einer Gesamtmenge von 0,3 bis 1,5 g/l zum Sichtbarmachen von Phosphatase-, Glukosidase- und Glukuronidase-Aktivität, ausgewählt aus p-Nitrophenylphosphat-dinatriumsalz, 5-Brom-4-chlor-3-indolyl-βD-glucopyranosid, 4-Methylumbelliferyl-βDglucopyranosid, 6-Chlor-3-indolyl-βD-glucopyranosid, 5-Brom-4-chlor-3-indolyl-βD-glucuronid-cyclohexylammoniumsalz, 5-Brom-6-chlor-3-indolyl-βD-glucuronid-cyclohexylammoniumsalz und 4-Methylumbelliferyl-βD-glucuronid-Trihydrat, und ein Gemisch von indikativen Verbindungen der Deaminase-Aktivität in einer Gesamtmenge von 1 bis 3 g/l, wobei das Gemisch aus dreiwertigen Metallsalzen und aromatischen Aminosäuren besteht, enthält.

2. Kulturmedium gemäß Anspruch 1, **dadurch gekennzeichnet, dass** es Nährstöffgrundlagen mit Proteinursprung in den folgenden Mengen im Medium enthält:
• Rinderbluthydrolysat 5-10 g/l
• Rinderherzextrakt 1-12 g/l
• enzymatisches Hydrolysat von Rinderherzmuskel 1-5 g/l
• enzymatisches Hydrolysat von Milchprotein 5-20 g/l
• enzymatisches Hydrolysat von Sojabohnenprotein 2-15 g/l
• enzymatisches Hydrolysat von Tiergewebe 1-5 g/l
• Saccharomyces cerevisiae-Hefe-Autolysat oder -Hydrolysat 2-15 g/l.

3. Kulturmedium gemäß Anspruch 1, wobei die Inhibitoren von Gram-negativen Mikroorganismen in den folgenden Mengen in dem Medium vorliegen:
• Thalliumacetat 0,55-1,6 g/l
• Nalidixinsäure 0,005-0,030 g/l.

4. Kulturmedium gemäß Anspruch 1, **dadurch gekennzeichnet, dass** es ein Gemisch von abbaubaren chromogenen und fluorogenen Substanzen für wenigstens einen der zu identifizierenden Organismen, ausgewählt in den folgenden Mengen, in dem Medium enthält:
• p-Nitrophenylphosphat-dinatriumsalz (PNP) 0,2 bis 0,8 g/l
• 5-Brom-4-chlor-3-indolyl-βD-glucopyranosid (x-glu), 4-Methylumbelliferyl-βD-glucopyranosid (Mu-glu), 6-Chlor- 3-indolyl-βD-glucopyranosid (Salmon-glu) 0,05 bis 0,4 g/l
• 5-Brom-4-chlor-3-indolyl-βD-glucuronid-cyclohexylammonium- Salz (x-gluc), 5-Brom-6-chlor-3-indolyl-βD-glucuronid- cyclohexylammoniumsalz (Magenta-Gluc) und 4-Methyl- umbelliferyl-βD-glucuronid-Trihydrat (MUG) 0,05 bis 0,2 g/l.

5. Kulturmedium gemäß Anspruch 1, **dadurch gekennzeichnet, dass** es ein Gemisch von indikativen Verbindungen der Deaminase-Aktivität in den folgenden Mengen in dem Medium enthält:
• trivalente Metallsalze, vorzugsweise Eisen(III)- Ammoniumcitrat in Mengen von 0,5 bis 1,0 g/l
• aromatische Aminosäuren, vorzugsweise Tryptophan und Phe- nylalanin, in Mengen von 1 bis 2 g/l.

6. Kulturmedium gemäß Anspruch 1, **gekennzeichnet durch** ein Gemisch von abbaubaren chromogenen und fluorogenen Substanzen zum Sichtbarmachen von Phosphatase-, Glucosidase- und Glucoronidaseaktivität in solchen Kombinationen, dass das Auftreten von Farben oder unterschiedlicher Fluoreszenz für jede Spezies garantiert ist, ausgewählt aus der Gruppe, bestehend aus:
a) PNP + x-glu + Magenta-gluc oder MUG
b) PNP + Salmon-glu + x-gluc oder MUG
c) PNP + Mu-glu + Magenta-gluc oder x-gluc.

7. Kulturmedium gemäß Anspruch 1, **dadurch gekennzeichnet, dass** es auch Agar mit einer Gelstärke von zwischen 400 und 700 g/cm² in Mengen von 10 und 14 g/l für die Präparation enthält.

8. Kulturmedium aus festem Medium gemäß Anspruch 1, wobei der pH-Wert von 7 bis 7,4 ist.

9. Kulturmedium aus festem Medium gemäß Anspruch 1, wobei seine Konzentration in Wasser von 25 bis 80 g/l variiert.

## Revendications

1. Milieu de culture sélectif destiné à l'isolation et à la détection précoce d'espèces du genre *Streptococcus,* **caractérisé** de façon à contenir un mélange de bases nutritives d'origine protéique dans une quantité totale de 15 à 58 g/l et ayant une teneur totale en azote comprise entre 10 et 14 % ; sélectionnées parmi un hydrolysat de sang bovin, un extrait de coeur de boeuf, un hydrolysat enzymatique de muscle de coeur de boeuf, un hydrolysat enzymatique de protéine du lait, un hydrolysat enzymatique de protéine de soja, un hydrolysat enzymatique de tissu animal, un autolysat ou un hydrolysat de levure *Saccharomyces cerevisiae* ; un mélange d'inhibiteurs de microorganismes Gram-négatifs dans une quantité totale de 0,55 à 1,63 g/l, où ledit mélange se compose d'acétate de thallium et d'acide nalidixique ; un mélange de substances chromogènes et fluorogènes dégradables dans une quantité totale de 0,3 à 1,5 g/l pour révéler l'activité de la phosphatase, de la glucosidase et de la glucuronidase, sélectionnées parmi un sel disodique de p-nitrophénylphosphate, le 5-bromo-4-chloro-3-indolyl-βD-glucopyranoside, le 4-méthylombelliféryl-βD-glucopyranoside, le 6-chloro-3-indolyl-βD-glucopyranoside, le sel cyclohexylammonium de 5-bromo-4-chloro-3-indolyl-βD-glucuronide, le sel cyclohexylammonium de 5-bromo-6-chloro-3-indolyl-βD-glucuronid, et le trihydrate de 4-méthylombelliferyl-βD-glucuronide ; et d'un mélange de composés indicateurs de l'activité de la désaminase, dans une quantité totale de 1 à 3 g/l, où ledit mélange se compose de sels métalliques trivalents et d'acides aminés aromatiques.

2. Milieu de culture selon la revendication 1, **caractérisé** de façon à contenir des bases nutritives d'origine protéique dans les quantités suivantes au sein du milieu :
hydrolysat de sang bovin : de 5 à 10 g/l,
extrait de coeur de boeuf : de 1 à 12 g/l,
hydrolysat enzymatique de muscle de coeur de boeuf : de 1 à 5 g/l,
hydrolysat enzymatique de protéine du lait : de 5 à 20 g/l,
hydrolysat enzymatique de protéine de soja : de 2 à 15 g/l
hydrolysat enzymatique de tissu animal : de 1 à 5 g/l;
autolysat ou hydrolysat de levure *Saccharomyces cerevisiae :* de 2 à 15 g/l.

3. Milieu de culture selon la revendication 1, dans lequel les inhibiteurs de microorganismes Gram-négatifs sont présents dans les quantités suivantes au sein du milieu :
acétate de thallium : de 0,55 à 1,6 g/l,
acide nalidixique : de 0,005 à 0,030 g/l.

4. Milieu de culture selon la revendication 1, **caractérisé** de façon à contenir un mélange de substances chromogènes et fluorogènes dégradables pour au moins un des organismes à identifier, sélectionnées dans les quantités suivantes au sein du milieu :
sel disodique de p-nitrophénylphosphate (PNP) : de 0,2 à 0,8 g/l,
5-bromo-4-chloro-3-indolyl-βD-glucopyranoside (x-glu), 4-méthylombelliféryl-βD-glucopyranoside (Mu-glu), 6-chloro-3-indolyl-βD-glucopyranoside (Salmon-glu) : de 0,05 à 0,4 g/l,
sel cyclohexylammonium de 5-bromo-4-chloro-3-indolyl-βD-glucuronide (x-gluc), sel cyclohexylammonium de 5-bromo-6-chloro-3-indolyl-βD-glucuronide (Magenta-gluc) et trihydrate de 4-méthylombelliféryl-βD-glucuronide (MUG) : de 0,05 à 0,2 g/l.

5. Milieu de culture selon la revendication 1, **caractérisé** de façon à contenir un mélange de composés indicateurs de l'activité de la désaminase dans les quantités suivantes au sein du milieu :
sels métalliques trivalents, de préférence citrate d'ammonium ferrique, dans des quantités de 0,5 à 1,0 g/l,
acides aminés aromatiques, de préférence tryptophane et phénylalanine, dans des quantités de 1 à 2 g/l.

6. Milieu de culture selon la revendication 1, **caractérisé par** un mélange de substances chromogènes et fluorogènes dégradables pour révéler l'activité de la phosphatase, de la glucosidase et de la glucuronidase, dans des combinaisons telles qu'elles garantissent l'apparition de couleurs ou d'une fluorescence différente(s) pour chaque espèce, sélectionnées dans le groupe composé de
a) PNP + x-glu + Magénta-gluc ou MUG
b) PNP + Salmon-glu + x-gluc ou MUG
c) PNP + Mu-glu + Magenta-gluc ou x-gluc.

7. Milieu de culture selon la revendication 1, **caractérisé** pour contenir également de l'agar-agar ayant une force de gélification comprise entre 400 et 700 g/cm² dans des quantités de 10 à 14 g/l pour la préparation d'un milieu solide.

8. Milieu de culture selon la revendication 1, dans lequel la valeur de pH est comprise entre 7 et 7,4.

9. Milieu de culture selon la revendication 1, dans lequel sa concentration dans l'eau varie de 25 à 80 g/l.
